# EUROPEAN PATENT APPLICATION

(11) **EP 4 406 560 A1**
(43) Date of publication of application: **31.07.2024**
(21) Application number: 24153871.9
(22) Date of filing: 25.01.2024
(51) Int. Cl.: A61L 2/22

(54) **CONTAINER-STERILIZER**

(30) Priority: 25.01.2023 JP 2023009246
(71) Applicant: Shibuya Corporation, Kanazawa-shi, Ishikawa 920-8681 (JP)
(72) Inventor: NISHINO, Yukinobu, Kanazawa-shi, Ishikawa, 920-8681 (JP); KAYA, Koji, Kanazawa-shi, Ishikawa, 920-8681 (JP)
(74) Representative: Hoffmann Eitle

(57) **Abstract**

A container-sterilizer includes a rotating body and a manifold which are configured to rotate coaxially and integrally with a rotational shaft which is rotatably supported by a fixed member. Sterilizing nozzles to inject a sterilizing agent are connected to the manifold. A penetrating member attached to the fixed member is provided in the rotational shaft to pass through the rotational shaft to the manifold. The manifold has supply openings communicating with the sterilizing nozzles. The penetrating member has a shielding member which is provided in the manifold and which is configured to supply the sterilizing agent in the manifold to some supply openings among the plurality of sterilizing nozzles.

## Description

### BACKGROUND OF THE INVENTION

### 1. Field of the Invention

The present invention relates to a container-sterilizer.

### 2. Description of the Related Art

Conventionally, there is known a device for sterilizing the inside of a resin container with a sterilizing agent such as hydrogen peroxide gas, as disclosed in Japanese Patent Publication Nos. 4700946, 4526820, and 6819709. In a device described in `946, a container passes under a nozzle disposed at a predetermined position where a sterilizing agent is injected onto an inner surface of the container. However, in such a construction, in which the sterilizing agent is supplied into the container only when the container passes directly under the nozzle, the period of time when the container passes directly under the nozzle becomes shorter as the container is conveyed at a higher speed. Thus, for carrying out an adequate sterilization, it is necessary to increase the number of nozzles. Furthermore, there is a problem of excess discharge of the sterilizing agent, since the sterilizing agent is continuously discharged from the nozzle, i.e., the sterilizing agent is discharged even when a container is not positioned directly under the nozzle.

On the other hand, a container-sterilizer described in '820 is configured such that a nozzle following a container injects a sterilizing agent into the container. In this device, however, the sterilizing agent is continuously discharged even in a section in which no container is present, which creates a problem in which excess discharge of the sterilizing agent reduces the sterilization efficiency.

'709 discloses a device, by which the above-described problems are solved. In the device, a supply unit supplying the sterilizing agent is provided with a fixed covering body, a fixed shielding plate and a rotating plate, so that the sterilizing agent is injected in a predetermined section. That is, the fixed shielding plate is disposed in a space formed between the fixed covering body and an upper surface of the rotating plate, and a nozzle injecting the sterilizing agent is provided on the bottom surface of the rotating plate. The fixed shielding plate is formed with an opening in a part corresponding to the predetermined section, such that the sterilizing agent is supplied in the fixed covering body before passing through the opening and ultimately entering a nozzle positioned within the section.

In the device disclosed by '709, a sealing member is attached to the outer periphery of the rotating plate for preventing the sterilizing agent from leaking out between the fixed covering body and the rotating plate. However, the sealing member is provided along the whole periphery of the rotating plate, which has a long circumference and high peripheral speed. Therefore, a problem exists because a large load applied to the sealing member could cause the sealing member to deteriorate rapidly. On the other hand, a clearance of a certain size is needed between the fixed shielding plate and the rotating plate, and the rotating plate has a large surface area. Thus, a relatively large amount of sterilizing agent could collect between the fixed shielding plate and the rotating plate, and the sterilizing agent may leak into nozzles outside of the predetermined section.

### SUMMARY OF THE INVENTION

Therefore, an object of the present invention is to provide a container-sterilizer in which the leakage of the sterilizing agent is prevented, so that the sterilizing agent can be efficiently supplied into the containers.

According to one aspect of the present invention, a container-sterilizer includes a fixed member, a rotational shaft rotatably supported by the fixed member, a rotating body configured to rotate coaxially and integrally with the rotational shaft, a manifold which is configured to rotate coaxially and integrally with the rotational shaft and to which a plurality of sterilizing nozzles to inject a sterilizing agent is connected, and a penetrating member attached to the fixed member and provided in the rotational shaft to pass through the rotational shaft to the manifold. The rotating body includes a plurality of holding members which is configured to hold containers. The manifold has a plurality of supply openings communicating with the plurality of sterilizing nozzles. The penetrating member has a shielding member which is provided in the manifold and which is configured to supply the sterilizing agent in the manifold to some supply openings among the plurality of sterilizing nozzles.

Preferably, the penetrating member comprises a sterilizing agent supply pipe that is configured to supply the sterilizing agent into the manifold.

Preferably, the shielding member is provided near the supply openings along an inner wall of the manifold.

According to the present invention, a container-sterilizer can be obtained, by which leakage of the sterilizing agent is prevented, so that the sterilizing agent can be efficiently supplied into the containers.

### BRIEF DESCRIPTION OF THE DRAWINGS

The object and advantages of the present invention will be better understood from the following description, with reference to the accompanying drawings in which:
Fig. 1 is a plane view showing a simplified diagram of an entire container conveying system provided with a container-sterilizer of an embodiment of the present invention;
Fig. 2 is a side view schematically showing the structure of the container-sterilizer;
Fig. 3 is a sectional view showing the structure of a combination of a rotary joint and a manifold;
Fig. 4 is a horizontal sectional view showing an inner structure of the manifold; and
Fig. 5 is a horizontal sectional view showing an inner structure of the manifold having a shielding member
different from that of Fig. 4.

### DESCRIPTION OF THE PREFERRED EMBODIMENTS

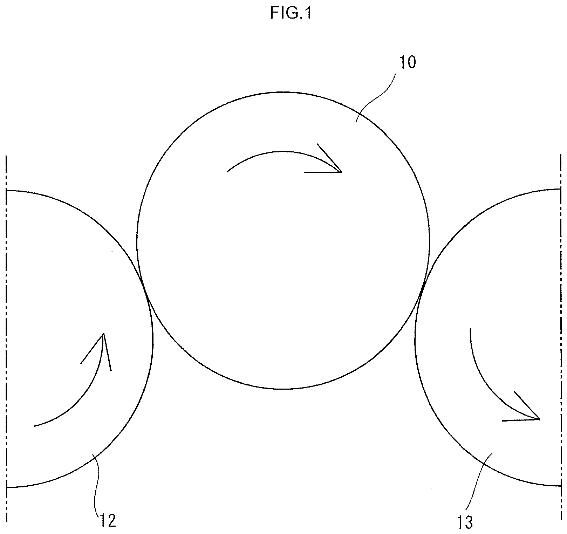

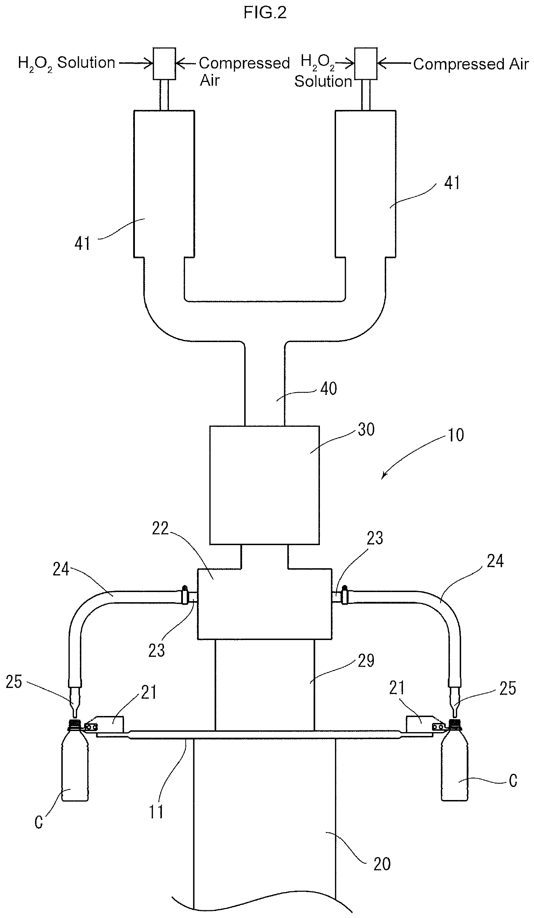

An embodiment of the present invention will be described below with references to the drawings. Fig. 1 shows a diagram of the whole complete structure of a container conveying system provided with a container-sterilizer 10 of an embodiment of the present invention. The container conveying system is configured to neck-convey a resin container, that is, to convey the container by holding onto its neck portion. Grippers, which are provided at predetermined intervals along the outer peripheries of the rotating body 11 (see Fig. 2) of the container-sterilizer 10, a supply wheel 12, and a discharge wheel 13, respectively, hold onto the neck portion of the container as it is conveyed along the circumferential direction. The grippers (not shown) of the supply wheel 12 and the discharge wheel 13 hold an upper portion of the flange formed on the neck portion of the container, and the grippers (holding members) 21 (see Fig. 2) of the container-sterilizer 10 hold a lower portion of the flange.

The container is transferred from the supply wheel 12 to the container-sterilizer 10, in which hydrogen peroxide gas (i.e., sterilizing agent) is injected into the container at a predetermined section where the container is conveyed by the rotating body 11, such that the inner surface of the container positioned in that particular section is sterilized. The container subjected to the sterilizing process is then transferred from the container-sterilizer 10 to the discharge wheel 13 and is conveyed to the next process.

Fig. 2 schematically shows a structure of the container-sterilizer 10. The rotating body 11 has a disk shape and is fixed at an upper end of a lower rotational shaft 20, which is driven by a drive motor (not shown) to rotate around the shaft center. The grippers 21 are provided at the peripheral edge of the rotating body 11 at predetermined intervals. Each gripper is always urged in the closing direction by a spring, and when a container C is supplied from the supply wheel 12 to the container-sterilizer 10, the container C conveyed by the supply wheel 12 is pressed against the gripper 21, which is forcibly opened to hold onto a lower portion of the flange of the neck portion of the container C. When the container C is transferred from the container-sterilizer 10 to the discharge wheel 13, the gripper of the discharge wheel 13 holds an upper portion of the flange of the container C held by the gripper 21. By maintaining this condition, the rotating body 11 and the discharge wheel 13 are rotated, and the container C is forcibly taken out and transferred to the discharge wheel 13.

A manifold 22 is provided above the rotating body 11, and connecting pipes 23 are provided at predetermined intervals on a cylindrical outer surface of the manifold. A base end of a sterilizing agent discharge pipe 24 is connected to the connecting pipe 23, and a sterilizing nozzle 25 is attached to a tip end of the sterilizing agent discharge pipe 24. The sterilizing nozzle 25 discharges hydrogen peroxide gas into the container C and is disposed above the container C held by the gripper 21. The sterilizing agent discharge pipe 24 is a metallic rigid body, for example, and a portion connected to the connecting pipe 23 extends horizontally, while a portion attached to the sterilizing nozzle 25, extends vertically. The manifold 22 and the rotating body 11 are connected by a cylindrical connecting member 29, such that the manifold 22 and the rotating body 11 rotate integrally, and the sterilizing nozzle 25 rotates to preserve the condition in which the sterilizing nozzle 25 faces the opening of the container C.

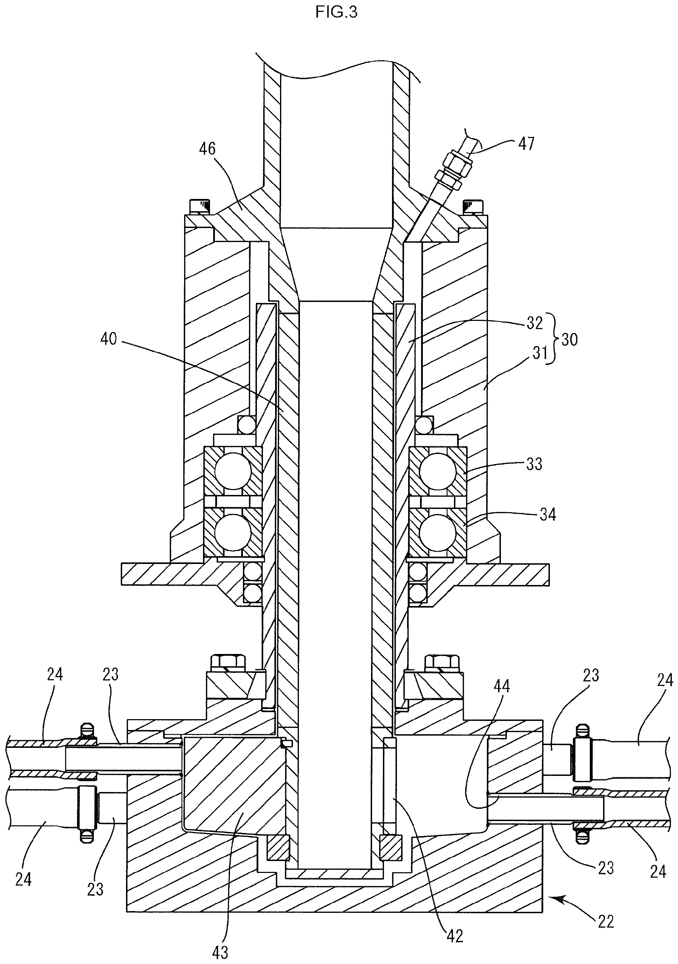

A rotary joint 30 is provided above the manifold 22, and a sterilizing agent supply pipe (i.e., penetrating member) 40 extends through the rotary joint 30. As described in detail with reference to Fig. 3, a lower end portion of the sterilizing agent supply pipe 40 projects from the rotary joint 30 and extends into the manifold 22. An upper end of the sterilizing agent supply pipe 40 is branched into two, and each end is connected to a heater 41. Hydrogen peroxide solution and compressed air are supplied to the heaters 41 and are mixed and heated to generate hydrogen peroxide gas. That is, the sterilizing agent supply pipe 40 generates hydrogen peroxide gas and supplies it into the manifold 22.

With reference to Fig. 3, the structures of the rotary joint 30 and the manifold 22 are described. The rotary joint 30 has a fixed member 31 and an upper rotational shaft 32, which is rotatably supported by an inner surface of the fixed member 31 through bearings 33 and 34. The manifold 22 is fixed to the lower end of the upper rotational shaft 32 and is coaxially and integrally rotatable with the upper rotational shaft 32. The sterilizing agent supply pipe 40 extends through the inside of the upper rotational shaft 32. A flange 46 formed on the sterilizing agent supply pipe 40 is attached to the upper end of the fixed member 31, and a washing water supply pipe 47 for supplying washing water into the rotary joint 30 is attached to the flange 46. The lower end of the sterilizing agent supply pipe 40 projects from the upper rotational shaft 32 to face the inside of the manifold 22. In the manifold 22, an injection port 42 is formed in the sterilizing agent supply pipe 40, and a shielding member 43 is provided on the opposite side of the injection port 42.

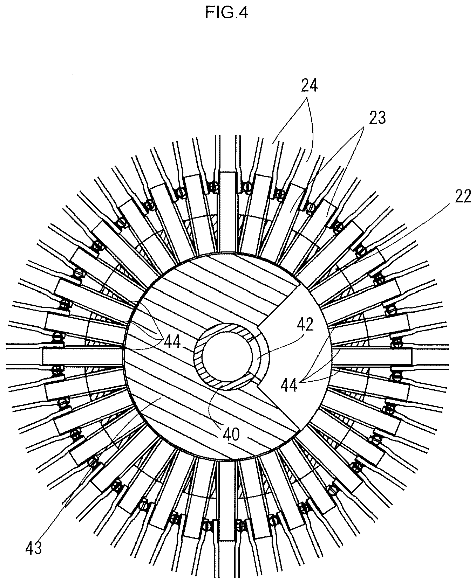

Fig. 4 is a horizontal sectional view showing an inner structure of the manifold 22. As shown in this figure, a plurality of supply openings 44 are formed in an outer peripheral wall of the manifold 22, and the radially extending connecting pipe 23 is mounted to each of the supply openings 44. As described above, the sterilizing agent discharge pipe 24 is connected to each connecting pipe 23, and thus the supply openings 44 are in communication with the sterilizing nozzles 25. Although the supply openings 44 are formed over the entire circumference of the manifold 22, as described above, the device is configured so that the injection port 42 of the sterilizing agent supply pipe 40 faces only a part of the supply openings 44 that correspond to a predetermined section where containers are positioned to receive hydrogen peroxide gas. That is, the shielding member 43 blocks or shields the other predetermined supply openings 44 so that the hydrogen peroxide gas discharged from the sterilizing agent supply pipe 40 is not supplied in areas where containers are not positioned to receive it. The shielding member 43 is provided near the supply openings 44 along an inner wall of the manifold 22, so that hydrogen peroxide gas in the manifold 22 does not leak into the predetermined supply openings 44.

An operation of the embodiment will be described below.

A container C transferred from the supply wheel 12 to the container-sterilizer 10 is held by the gripper 21 and rotary-conveyed to the discharge wheel 13 by the rotating body 11. In the rotary-conveyance, the manifold 22 coaxially and integrally rotates with the rotating body 11. Therefore, the sterilizing nozzle 25 connected to the manifold 22 is always positioned above the upper portion of the container C held by the gripper 21. In the manifold 22, the injection port 42 of the sterilizing agent supply pipe 40 faces the supply opening 44 corresponding to the container C located in the predetermined section to supply hydrogen peroxide gas, which is injected from the sterilizing nozzle 25 into the container C. Conversely, hydrogen peroxide gas is neither supplied to the supply opening 44 corresponding to another container that is not located in the predetermined section, nor supplied to the supply opening 44 corresponding to a section in which no container exists, since the supply openings 44 are blocked by the shielding member 43.

As described above and according to the embodiment, except for the supply openings 44 corresponding to the predetermined section in which a sterilizing process is performed, all other supply openings are blocked by the shielding member 43, and thus hydrogen peroxide gas does not pass between the shielding member 43 and an inner wall of the manifold 22 to leak into any sterilizing nozzle 25 that are not positioned in the predetermined section. Therefore, hydrogen peroxide gas is efficiently supplied to the containers positioned in the predetermined section, and due to this the sterilizing efficiency is improved.

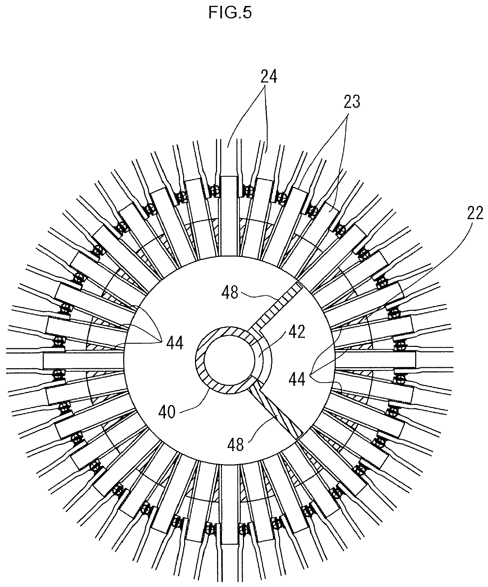

Fig. 5 shows an inner structure of the manifold 22 having the other shielding member (i.e., a shielding member 48). The difference from the shielding member 43 is that the shielding member 48 comprises a pair of plate members separating the supply opening 44 facing the injection port 42 from the other supply opening 44 not facing the injection port 42. That is, the shielding member 48 extends from the sterilizing agent supply pipe 40 in the radial direction of the manifold 22 and the tip portion of the shielding member 48 is close to an inner wall of the manifold 22, such that hydrogen peroxide gas injected from the injection port 42 is supplied only to the supply opening 44 corresponding to the predetermined section in which the sterilizing process is performed. Thus, due to this shielding member 48, the same effect of the embodiment comprising the shielding member 43 can be obtained.

According to the embodiments of the container-sterilizer, the leakage of the sterilizing agent is prevented, so that the sterilizing agent can be efficiently supplied into the containers.

It should be noted that the injection port 42 of the sterilizing agent supply pipe 40 does not need to face the supply opening 44 and may inject hydrogen peroxide gas through a bottom surface of the manifold 22, for example.

Furthermore, the lower end of the sterilizing agent supply pipe 40 is not necessarily positioned in the manifold and may be set close to the upper end of the rotational shaft 32 of the rotary joint 30. That is, it is possible to adopt a structure in which the shielding member 43 and a lower end surface of the sterilizing agent supply pipe 40 are connected by a penetrating member extending through the inside of the rotational shaft 32.

Although the embodiments of the present invention have been described herein with reference to the accompanying drawings, obviously many modifications and changes may be made by those skilled in this art without departing from the scope of the invention.

The present disclosure relates to subject matter contained in Japanese Patent Application No. 2023-009246 (filed on January 25, 2022) which is expressly incorporated herein, by reference, in its entirety.

## Claims

1. A container-sterilizer comprising:
a fixed member;
a rotational shaft rotatably supported by the fixed member;
a rotating body configured to rotate coaxially and integrally with the rotational shaft, the rotating body including a plurality of holding members which is configured to hold containers;
a manifold which is configured to rotate coaxially and integrally with the rotational shaft and to which a plurality of sterilizing nozzles to inject a sterilizing agent is connected, the manifold having a plurality of supply openings communicating with the plurality of sterilizing nozzles; and
a penetrating member attached to the fixed member and provided in the rotational shaft to pass through the rotational shaft to the manifold, the penetrating member having a shielding member which is provided in the manifold and which is configured to supply the sterilizing agent in the manifold to some supply openings among the plurality of sterilizing nozzles.

2. The container-sterilizer according to claim 1, wherein the penetrating member comprises a sterilizing agent supply pipe that is configured to supply the sterilizing agent into the manifold.

3. The container-sterilizer according to claim 1 or claim 2, wherein the shielding member is provided near the supply openings along an inner wall of the manifold.
